# EUROPEAN PATENT APPLICATION

(11) **EP 3 851 125 A1**
(43) Date of publication of application: **21.07.2021**
(21) Application number: 19852722.8
(22) Date of filing: 21.08.2019
(51) Int. Cl.: A61K 45/00, A61K 31/7088, A61K 31/7105, A61K 31/713, A61P 35/04, A61P 43/00

(54) **AGENT USING HSP47 INHIBITOR TO SUPPRESS METASTASIS**

(30) Priority: 22.08.2018 JP 2018155148
(71) Applicant: Nitto Denko Corporation, Ibaraki-shi, Osaka 567-8680 (JP)
(72) Inventor: YONEDA, Akihiro, Sapporo-shi, Hokkaido 160-0808 (JP); TAMURA, Yasuaki, Sapporo-shi, Hokkaido 060-0808 (JP); TAKEI, Norio, Sapporo-shi, Hokkaido 060-0808 (JP); SAWADA, Kaori, Sapporo-shi, Hokkaido 060-0808 (JP)
(74) Representative: Hoffmann Eitle
(86) International application number: PCT/JP2019/032614
(87) International publication number: WO 2020/040186

(57) **Abstract**

Provided is a metastasis suppressing agent containing a HSP47 inhibitor.

## Description

### [Technical Field]

The present invention relates to a medicine for suppressing metastasis of cancer and a method therefor.

### [Background Art]

With regard to cancer therapy, therapy by inducing cancer cell death, therapy by suppressing proliferation of cancer cells, and therapy by eliminating cancer tissues constitute the mainstream therapy; however, it is important to implement therapy of suppressing metastasis of cancer cells in combination with these therapies, from the viewpoint of preventing progression of cancer or recurrence of cancer. However, a therapy effective for suppressing metastasis of cancer cells has not been realized. Therefore, with regard to cancer therapy, it is desirable to establish a technique for suppressing metastasis of cancer cells. Regarding a technique for cancer therapy, it is described in Patent Document 1 that a tumor is treated by suppressing growth of the tumor by using a molecule that targets heat shock protein 47 (HSP47).

Triple-negative breast cancer is negative for expression of estrogen receptor (ER), progesterone receptor (PgR), and human epidermal growth factor receptor 2 (HER2), and has high metastatic potential, so that the effect of conventional breast cancer treatment methods (chemotherapy, hormone therapy, and molecular targeted therapy) is hardly exhibited, and currently, there is no solution to that situation. For example, in recent years, antibody medicines targeting HER2 have been used as molecular targeted drugs for breast cancer therapy; however, since triple negative breast cancer is HER2-negative, it is difficult to induce cell death and proliferation suppression by means of such molecular targeted drugs. Furthermore, triple-negative breast cancer does not respond to hormone therapy. Treatment of surgically removing triple-negative breast cancer is possible; however, even if cancerous tissue is surgically removed, some of cancer cells may not be completely removed from the body. Since triple-negative breast cancer has a very high ability to metastasize to distant tissues, even if surgical therapy has been performed, it is desirable to perform treatment to suppress metastasis as well, in consideration of the risk of remaining cancer. However, in general, no effective treatment for suppressing metastasis of cancer cells has been established. As such, the mechanism for acquiring high metastatic potential for breast cancer, especially triple-negative breast cancer, is not clearly known, and there is no treatment method capable of controlling and suppressing metastasis.

### [Citation List]

Patent Document 1: U.S. Patent Publication No. 2017/0218365

### [Summary of the Invention]

### Problems to be solved by the Invention

It is an object of the invention to suppress metastasis of a cancer with high malignancy and high metastatic potential.

### Means for Solving the Problems

The inventors of this invention repeatedly conducted a thorough invention in order to address the above-described problems, and the inventors found that in triple-negative breast cancer, some of the cell populations highly express HSP47, HSP47-positive triple-negative breast cancer cells have very high metastatic potential, and in triple-negative human breast cancer, the metastatic potential can be suppressed by suppressing the expression of HSP47, thus completing the invention.

Furthermore, the inventors newly found that HSP47 protein binds to non-muscle myosin IIA; NMIIA (MYH9), which is an important factor of cell movement. HSP47 binds to a factor important for cell movement and is involved in cell movement control, while an HSP47 inhibitor is expected to suppress cell movement through inhibition of the expression of HSP47. Since MYH9 is expressed not only in triple-negative human breast cancer but also in cancer cells other than breast cancer, cell movement regulation through regulation of the expression of HSP47 is expected to be applicable as well to cancer cells other than triple-negative human breast cancer.

That is, the invention relates to the following.
(1) A cancer metastasis suppressant comprising an HSP47 inhibitor.
(2) The cancer metastasis suppressant according to (1), wherein the cancer is breast cancer.
(3) The cancer metastasis suppressant according to (2), wherein the cancer is triple-negative breast cancer negative for hormone receptors and negative for human epidermal growth factor receptor 2 (HER2).
(4) The cancer metastasis suppressant according to (3), wherein the hormone receptors are an estrogen receptor and a progesterone receptor.
(5) The cancer metastasis suppressant according to (3) or (4), wherein the cancer cell is a triple-negative breast cancer cell expressing MYH9 in addition to HSP47.
(6) The cancer metastasis suppressant according to any one of (1) to (5), wherein the HSP47 inhibitor is an interfering nucleic acid against HSP47, a ribozyme, an antisense nucleic acid, a microRNA, a short hairpin RNA, a vector expressing any of these, or a cell transformed with any of these.
(7) A pharmaceutical composition comprising the cancer metastasis suppressant according to any one of (1) to (6).
(8) The pharmaceutical composition according to (7), wherein the pharmaceutical composition is intended for treating cancer.
(9) The pharmaceutical composition according to (8), wherein the pharmaceutical composition is intended for treating metastatic lung cancer.
(10) Use of an HSP47 inhibitor in the manufacture of a medicine for suppressing metastasis of cancer.
(11) The use according to (10), wherein the cancer is triple-negative breast cancer.
(12) A composition for use in the suppression of metastasis of cancer, comprising an HSP47 inhibitor.
(13) The composition according to (12), wherein the cancer is triple-negative breast cancer.
(14) A method for suppressing metastasis of cancer, comprising administering an effective amount of an HSP47 inhibitor to a cancer patient.
(15) The method for suppressing metastasis of cancer according to (14), wherein the cancer patient is a breast cancer patient, and the method comprises a step of checking whether the breast cancer patient is a triple-negative breast cancer patient before administering the effective amount of the HSP47 inhibitor.
(16) The method for suppressing metastasis of cancer according to (14) or (15), comprising a step of checking whether the cancer tissue of the cancer patient expresses MYH9 in addition to HSP47, before administering the effective amount of the HSP47 inhibitor.
(17) The method for suppressing metastasis of cancer according to any one of (14) to (16), wherein the method is carried out together with a treatment selected from the group consisting of surgical therapy, radiation therapy, particle beam therapy, chemotherapy, and molecular targeted treatment.
(18) A method for suppressing metastasis of cancer in vitro, comprising treating cancer patient-derived cancer cells in vitro using an effective amount of an HSP47 inhibitor.

### Advantageous Effects of the Invention

The cancer metastasis suppressant of the invention can suppress metastasis of cancer by inhibiting the expression of HSP47 in cancer cells . The method of the invention can suppress metastasis of cancer by administering an HSP47 inhibitor.

### [Brief Description of the Drawings]

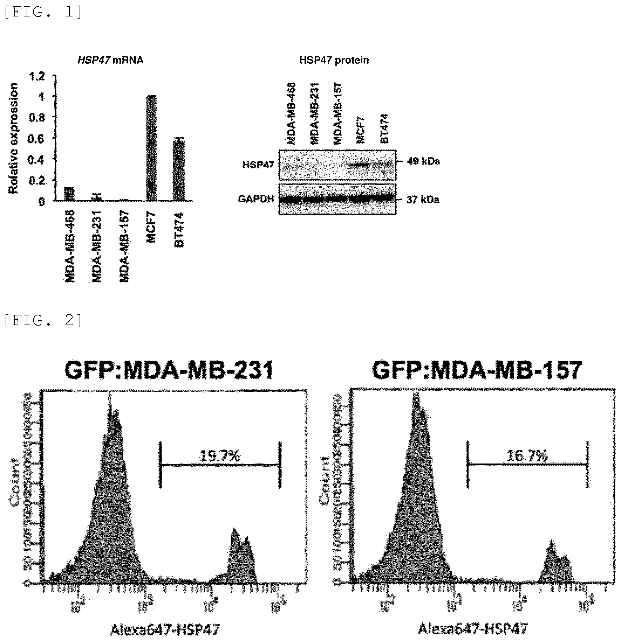

[FIG. 1] Fig. 1 shows the expression of HSP47 mRNA (left-side diagram) and HSP47 protein (right-side diagram) in human breast cancer cells. The expression of HSP47 mRNA and HSP47 protein in three kinds of triple-negative human breast cancer cells (MDA-MB-468, MDA-MB-231, and MDA-MB-157) and two kinds of non-triple-negative human breast cancer cells (MCF7 and BT474) was detected by Real time-PCR and a Western blotting method.
[FIG. 2] Fig. 2 shows the HSP47-positive cell rate in tumor-derived triple-negative human breast cancer cells. Two kinds of triple-negative human breast cancer cells (MDA-MB-231 and MDA-MB-157) were subcutaneously transplanted into Balb/c nu/nu mice at a concentration of 1×10⁶ cells/50 µL PBS, after tumor formation, tumor cells were harvested, and a flow cytometer analysis was performed using anti-HSP47 antibody (Enzo Life Sciences).

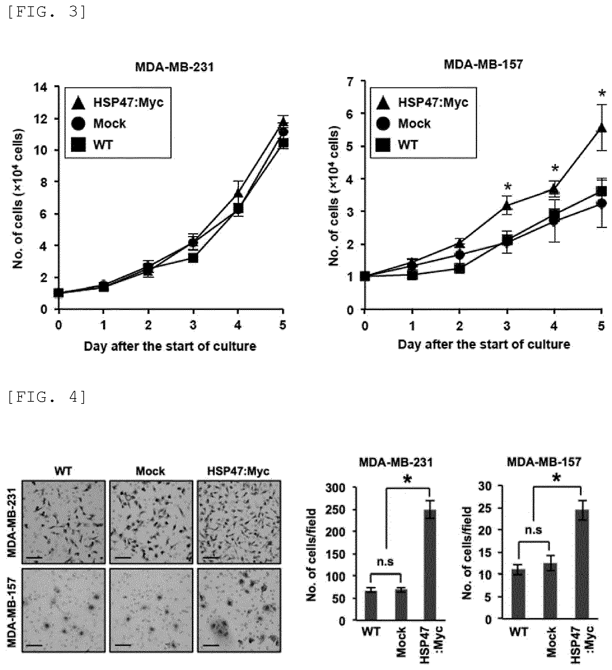

[FIG. 3] Fig. 3 shows the cellular proliferative capacity of HSP47 forced expression triple-negative human breast cancer cells. HSP47 forced expression triple negative human breast cancer cells (MDA-MB-231 and MDA-MB-157) created by gene transfer of pCMV6-entry plasmid (Mock) or pCMV6-HSP47-Myc plasmid (HSP47:Myc) were seeded on a 6-well plate at a concentration of 1×10⁴ cells/well, and for 5 days after the initiation of culturing, the cellular proliferative capacity was measured by cell counting.
[FIG. 4] Fig. 4 shows the cell motility of HSP47 forced expression triple-negative human breast cancer cells. HSP47 forced expression triple-negative human breast cancer cells (MDA-MB-231 and MDA-MB-157) created by gene transfer of pCMV6-entry plasmid (Mock) or pCMV6-HSP47-Myc plasmid (HSP47:Myc) were seeded in a double chamber at a concentration of 1×10⁴ cells/well, and on the 24^{th} hour after the initiation of culturing, hematoxylin staining was performed to measure the cell motility by cell counting.

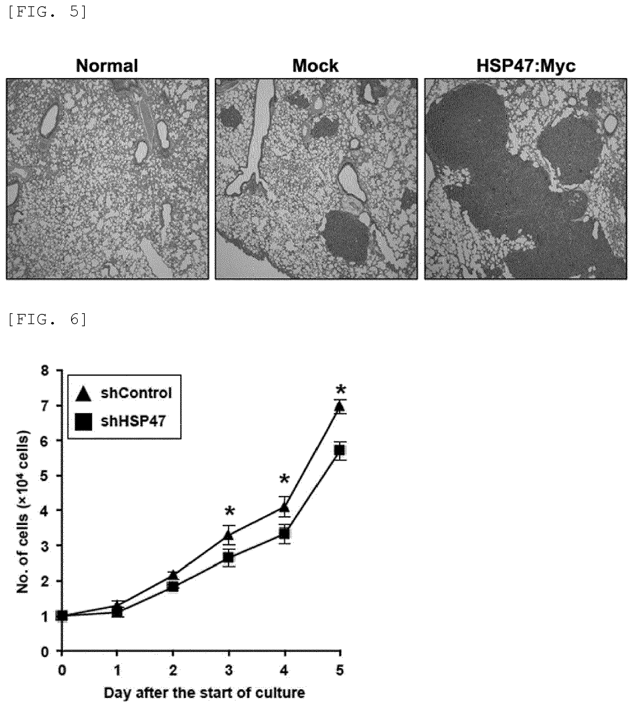

[FIG. 5] Fig. 5 shows the lung metastatic potential of HSP47 forced expression triple-negative human breast cancer cells. HSP47 forced expression triple-negative human breast cancer cells (MDA-MB-231) created by gene transfer of pCMV6-entry plasmid (Mock) or pCMV6-HSP47-Myc plasmid (HSP47:Myc) were subjected to caudal vein administration to Balb/c nu/nu mice at a concentration of 1×10⁶ cells/50 µL PBS, and metastatic potential to the lung was investigated by harvesting lung tissue on the 30^{th} day after cell administration and performing HE staining.
[FIG. 6] Fig. 6 shows the cellular proliferative capacity of triple-negative human breast cancer cells depending on suppression of HSP47 expression. Triple-negative human breast cancer cells (HSP47(+) MDA-MB-231 and HSP47(-) MDA-MB-231) created by gene transfer of Control shRNA expression plasmid (shControl) or HSP47 shRNA expression plasmid (shHSP47) were seeded on a 6-well plate at a concentration of 1×10⁴ cells/well, and for 5 days after the initiation of culturing, the cellular proliferative capacity was measured by cell counting.

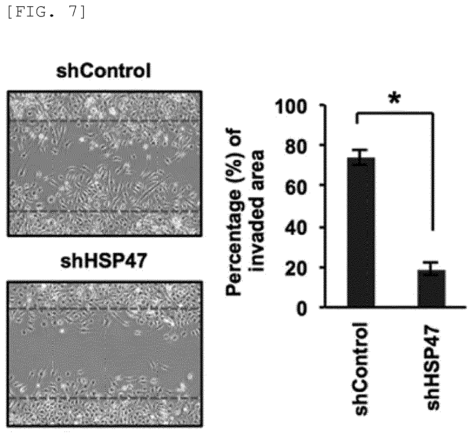

[FIG. 7] Fig. 7 shows the cell motility of triple-negative human breast cancer cells depending on suppression of HSP47 expression. Triple-negative human breast cancer cells (HSP47(+) MDA-MB-231 and HSP47(-) MDA-MB-231) created by gene transfer of Control shRNA expression plasmid (shControl) or HSP47 shRNA expression plasmid (shHSP47) were seeded on a 35-mm dish at a concentration of 1×10⁵ cells/well, and on the 24^{th} hour after the initiation of culturing, the cell motility was measured using a scratch assay.

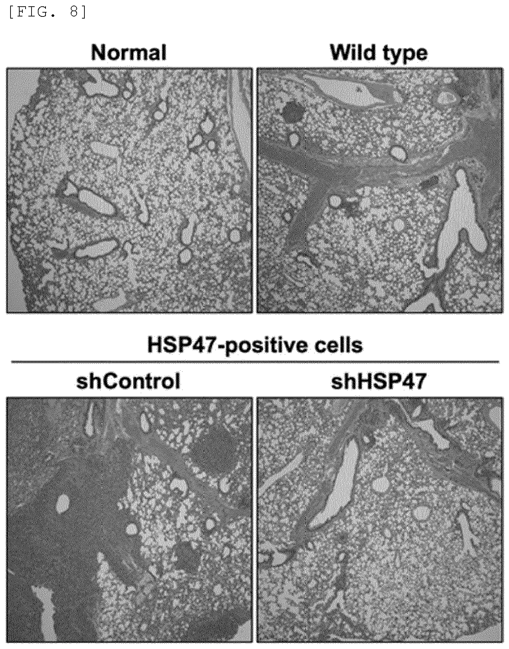

[FIG. 8] Fig. 8 shows the lung metastatic potential of human triple-negative breast cancer cells caused by suppression of HSP47 expression. Triple-negative human breast cancer cells (HSP47(+) MDA-MB-231 and HSP47(-) MDA-MB-231) created by gene transfer of Control shRNA expression plasmid (shControl) or HSP47 shRNA expression plasmid (shHSP47) were subjected to caudal vein administration to Balb/c nu/nu mice at a concentration of 1×10⁶ cells/50 µL PBS, and the metastatic potential to the lung was investigated by harvesting lung tissue on the 30^{th} day after cell administration and performing HE staining.

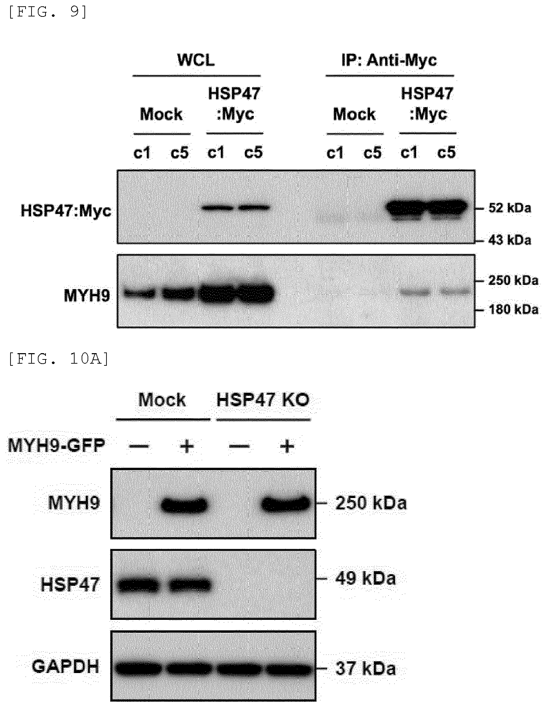

[FIG. 9] Fig. 9 shows an interaction between HSP47 and MYH9 in triple-negative breast cancer cells. A whole cell lysate (WCL) and a component extracted using an anti-Myc antibody-binding carrier were tested by SDS-PAGE, and association between MYH9 and HSP47 was confirmed by Western blotting.
[FIG. 10A] Fig. 10A shows expression of MYH9 protein and HSP47 protein in Mock cells, which belong to non-triple-negative breast cancer cell strain MCF7, MYH9-expressing cells (Mock + MYH9-GFP), HSP47 knockout (KO) cells, and MYH9-expressing HSP47 KO cells (HSP47 KO + MYH9-GFP).

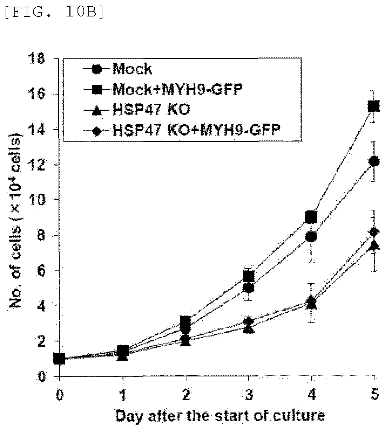

[FIG. 10B] Fig. 10B shows in vitro proliferation of Mock cells, MYH9-expressing cells (Mock + MYH9-GFP), HSP47 knockout (KO) cells, and MYH9-expressing HSP47 KO cells (HSP47 KO + MYH9-GFP) . *P < 0.05. n.s.: Not significant.

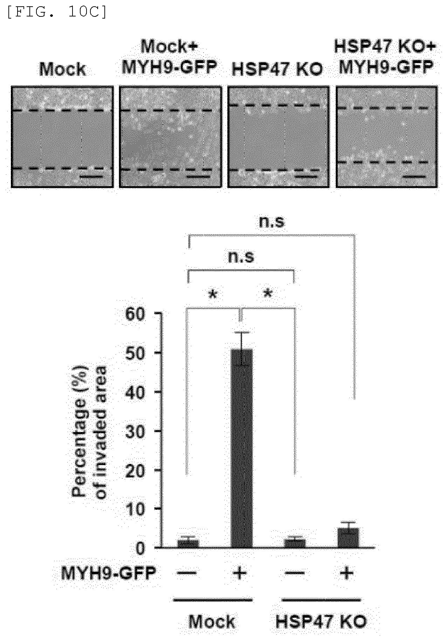

[FIG. 10C] Fig. 10C shows the results of investigating invasion of Mock cells, MYH9-expressing cells (Mock + MYH9-GFP), HSP47 knockout (KO) cells, and MYH9-expressing HSP47 KO cells (HSP47 KO + MYH9-GFP) by a scratch assay. Representative images of invaded regions (upper diagrams) and a quantification graph (lower diagram) are shown. *P < 0.05. n.s.: Not significant.

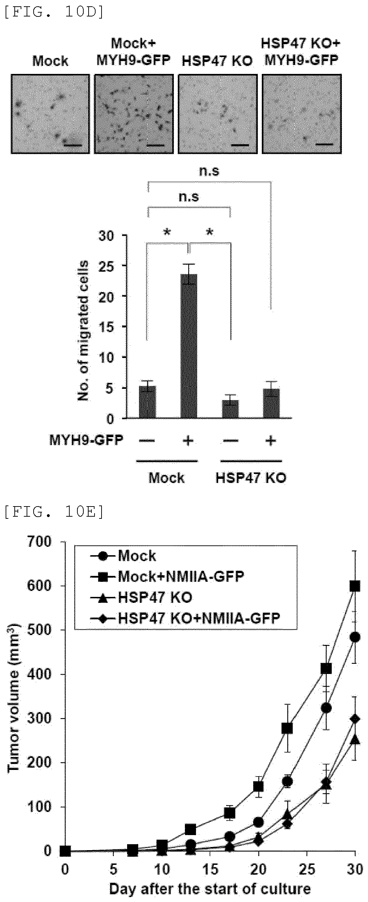

[FIG. 10D] Fig. 10D shows the results of investigating migration of Mock cells, MYH9-expressing cells (Mock + MYH9-GFP), HSP47 knockout (KO) cells, and MYH9-expressing HSP47 KO cells (HSP47 KO + MYH9-GFP) by a double chamber assay. Representative images of migrated cells (upper diagrams) and a quantification graph (lower diagram) are shown. *P < 0.05. n.s.: Not significant.
[FIG. 10E] Fig. 10E shows the results of investigating tumorigenic potential by transplanting Mock cells, MYH9-expressing cells (Mock + MYH9-GFP), HSP47 knockout (KO) cells, and MYH9-expressing HSP47 KO cells (HSP47 KO + MYH9-GFP) into the breast fat pads of female Balb/c nu/nu mice. *P < 0.05. n.s.: Not significant.

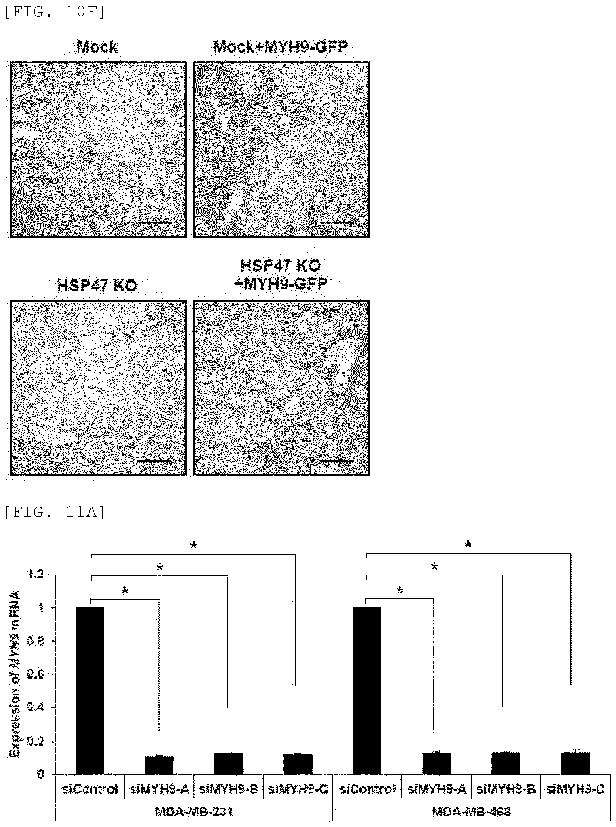

[FIG. 10F] Fig. 10F shows the results of a histological analysis of the lung tissue of female Balb/c nu/nu mice transplanted with Mock cells, MYH9-expressing cells (Mock + MYH9-GFP), HSP47 knockout (KO) cells, and MYH9-expressing HSP47 KO cells (HSP47 KO + MYH9-GFP). Scale bar: 200 µm.
[FIG. 11A] Fig. 11A shows expression of MYH9 mRNA in human breast cancer cells. Expression of MYH9 mRNA in two kinds of triple-negative human breast cancer cells (MDA-MB-231 and MDA-MB-468) was detected by Real time-PCR.

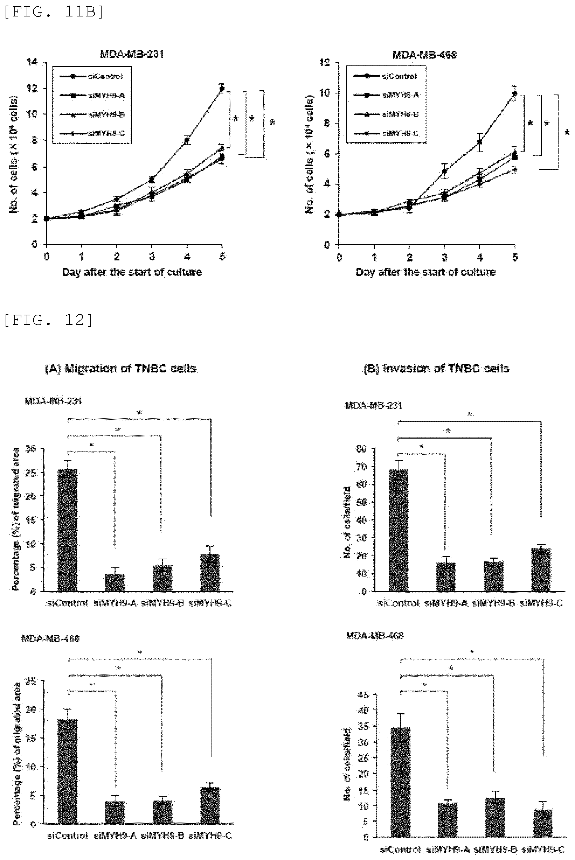

[FIG. 11B] Fig. 11B shows the cellular proliferative capacity of triple-negative human breast cancer cells depending on suppression of MYH9 expression. Triple-negative human breast cancer cells (MDA-MB-231 and MDA-MB-468) created by gene transfer of siControl or MYH9 siRNA (siMYH9-A, siMYH9-B, or siMYH9-C) were seeded on a 6-well plate at a concentration of 1×10⁴ cells/well, and for 5 days after the initiation of culturing, the cellular proliferative capacity was measured by cell counting.
[FIG. 12] Fig. 12A shows the cell motility of triple-negative human breast cancer cells depending on suppression of MYH9 expression. Triple-negative human breast cancer cells (MDA-MB-231 and DMA-MB-468) created by gene transfer of siControl or MYH9-siRNA (siMYH9-A, siMYH9-B, or siMYH9-C) were seeded on a 35-mm dish at a concentration of 1×10⁵ cells/well, and on the 24^{th} hour after the initiation of culturing, cell motility was measured using a scratch assay. Fig. 12B shows the lung metastatic potential of human triple-negative breast cancer cells depending on the suppression of HSP47 expression. Triple-negative human breast cancer cells (MDA-MB-221 and MDA-MB-468) created by gene transfer of siControl or MYH9-siRNA (siMYH9-A, siMYH9-B, or siMYH9-C) were cultured in a Transwell chamber at a concentration of 2×10⁴ cells/insert. After 24 hours from the initiation of culturing, cells that had passed through a Transwell membrane were immobilized with 4% paraformaldehyde and stained using hematoxylin. The results of the number of cells per field are shown in Fig. 12B.

### [Description of Embodiments]

Provided in the present specification are a cancer metastasis suppressant comprising an HSP47 inhibitor, a pharmaceutical composition and a kit comprising this suppressant, and a method for suppressing metastasis of cancer, the method comprising administering an HSP47 inhibitor.

The cancer metastasis suppressant, pharmaceutical composition, kit, and method of the invention have a feature of using a nucleic acid molecule (for example, a small interfering nucleic acid (siNA), a small interfering RNA (siRNA), a double-stranded RNA (dsRNA), a microRNA (miRNA), or a short hairpin RNA (shRNA)) that binds to transcript RNA (including pre-mRNA and mRNA) of HSP47, for example, mRNA of HSP47 as set forth in SEQ ID NO:1, or knocking out the HSP47 gene using a genome editing technology.

### 1. Cancer metastasis suppressant of invention

An aspect of the invention relates to a cancer metastasis suppressant comprising an HSP47 inhibitor, for example, a cancer metastasis suppressant comprising an inhibitory agent for the expression of HSP47.

The base sequence and amino acid sequence of the HSP47 gene are known in the pertinent art, and in this invention, the mRNA sequence of HSP47 is set forth in SEQ ID NO:1.

According to the invention, the transcript RNA of HSP47 may be, for example, derived from a human and may have a base sequence set forth in SEQ ID NO:1 or the same base sequence except that one to several nucleotides are deleted, substituted, added, or inserted, or a base sequence having a sequence identity of 70% or higher, 80% or higher, or 90% or higher, preferably 95% or higher, and more preferably 98% or higher or 99% or higher, with each of the above-described base sequences.

In the present specification, the term "several" means a number of bases of 2 to 10, preferably 2 to 5, and more preferably 2 or 3. Furthermore, the sequence identity can be determined using, for example, a known algorithm such as BLAST.

According to the invention, the inhibitory agent for the expression of HSP47 may be a component that inhibits the expression of HSP47 or a component that inhibits the functions of HSP47 protein, and examples include a siRNA or a precursor RNA thereof (for example, shRNA), which has RNA interference (RNAi) action, or any one of modified RNAs thereof, or alternatively, a vector containing a DNA encoding a siRNA against the transcript RNA of the HSP47 gene. Furthermore, according to the invention, the inhibitory agent for the expression of HSP47 may be, for example, a small interfering nucleic acid (siNA), a ribozyme, a shRNA, or a miRNA. The vector may contain an antisense RNA, an antisense DNA, a DNA encoding the antisense RNA, or an antisense DNA thereof. According to the invention, the inhibitory agent for the expression of HSP47 is preferably, for example, a shRNA or a plasmid vector containing a shRNA. Such a shRNA can be purchased from OriGene Technologies, Inc. Alternatively, according to the invention, as an inhibitory agent for the expression of HSP47, the HSP47 gene can be knocked out using a genome editing technology of using a plasmid encoding the gRNA sequence (for example, 5'-CAAGATGCGAGACGAGTTATAGG-3' (SEQ ID NO:8)).

According to the invention, the siRNA may be a double-stranded RNA composed of sense RNA and antisense RNA, the double-stranded RNA having an antisense RNA containing 18 to 25 nucleotides, preferably 20 to 24 nucleotides, and more preferably 21 to 23 nucleotides, which is substantially complementary to a portion of the transcript RNA of the HSP47 gene, and having RNAi (RNA interference) action.

According to the invention, the term "complementary" means that a nucleic acid can form hydrogen bonds with another nucleic acid sequence by the classical Watson-Crick type or other non-classical type.

Furthermore, according to the invention, the term "substantially complementary" includes not only a case where all the contiguous residues of a nucleic acid sequence form hydrogen bonds with the same number of contiguous residues in another nucleic acid sequence, but also a case where, for example, 70%, 80%, and 90% of residues among all the residues of a nucleic acid sequence form hydrogen bonds with the residues of another nucleic acid sequence.

Therefore, according to the invention, the siRNA may have an antisense RNA containing a nucleotide obtained by modifying several bases in a nucleotide that is 100% complementary to a portion of the transcript RNA of the HSP47 gene.

Furthermore, according to the invention, the 3'-termini of the sense RNA and the antisense RNA may each have a protruding end of 2 to 5 nucleotides, and preferably two nucleotides. According to the invention, the siRNA may be a modified siRNA.

For example, according to the invention, the siRNA may be a combination (siHSP47-A) of 5'-CUACGACGACGAGAAGGAAtt-3' (SEQ ID NO:2) of the sense strand and 5'-UUCCUUCUCGUCGUCGUAGta-3' (SEQ ID NO:3) of the antisense strand (Ambion), a combination (siHSP47-B) of 5'-AGCCCUCUUCUGACACUAAtt-3' (SEQ ID NO:4) of the sense strand and 5'-UUAGUGUCAGAAGAGGGCUgg-3' (SEQ ID NO:5) of the antisense strand (Ambion), or a combination (siHSP47-C) of 5'-GGACAGGCCUCUACAACUAtt-3' (SEQ ID NO:6) of the sense strand and 5'-UAGUUGUAGAGGCCUGUCCtt-3' (SEQ ID NO:7) of the antisense strand (created by Nitto Denko Corporation).

Furthermore, according to the invention, the cancer metastasis suppressant may also comprise, in addition to the HSP47 inhibitor, an inhibitor of MYH9 (for example, Accession No. NM_002473 (SEQ ID NO:15)), for example, a plasmid encoding the gRNA sequence, or alternatively, a MYH9 siRNA such as a combination (siMYH9-A) of 5'-GGGUAUCAAUGUGACCGAUtt-3' (SEQ ID NO:9) of the sense strand and 5'-AUCGGUCACAUUGAUACCCaa-3' (SEQ ID NO:10) of the antisense strand, a combination (siMYH9-B) of 5'-CCACCAACCUCACAGAAGAtt-3' (SEQ ID NO:11) of the sense strand and 5'-UCUUCUGUGAGGUUGGUGGtg-3' (SEQ ID NO:12) of the antisense strand, or a combination (siMYH9-C) of 5'-CGGCAAGGUGGAUUACAAAtt-3' (SEQ ID NO:13) of the sense strand and 5'-UUUGUAAUCCACCUUGCCGgc-3' (SEQ ID NO:14) of the antisense strand (created by Nitto Denko Corporation).

Regarding the introduction of an inhibitor of HSP47 or MYH9 into cells, examples of a known technique for introduction that can be used include, without limitations, a lipofectamine method, a lipofection method, a calcium phosphate method, an ultrasonic introduction method, an electroporation method, a particle gun method, a method of utilizing a viral vector (for example, an adenovirus vector, an adeno-associated virus vector, or a retrovirus vector), and a microinjection method.

In the case of using a viral vector, the viral titer may be 1×10³ to 1×10¹⁵ p.f.u. (plaque-forming unit), and the viral vector can be used at a viral titer of preferably 1×10⁵ to 1×10¹³, more preferably 1×10⁷ to 1×10¹¹, and even more preferably 1×10⁸ to 1×10¹⁰.

The nucleic acid molecule may be used as a naked nucleic acid or may be used after being incorporated into various nucleic acid constructs or vectors. Regarding the vectors, any known vectors such as a plasmid vector, a phage vector, a phagemid vector, a cosmid vector, and a viral vector can be utilized. It is preferable that the nucleic acid constructs or vectors include at least appropriate transcriptional or translational regulatory sequences derived from, for example, mammalian, microbial, viral, or insect genes. Such regulatory sequences include a sequence having a regulatory role for gene expression, for example, a transcription promoter or enhancer, an operator sequence for regulating transcription, a sequence encoding a ribosome binding site within messenger RNA, and a sequence appropriate for regulating transcription, translation initiation, or transcription termination.

### 2. Pharmaceutical composition of invention

The invention also relates to a pharmaceutical composition comprising the above-described cancer metastasis suppressant. Since the cancer metastasis suppressant is capable of reducing motility of cancer cells and suppressing the metastasis of cancer, the cancer metastasis suppressant is useful as an active ingredient of a pharmaceutical composition. The pharmaceutical composition according to the invention may comprise the above-described HSP47 inhibitor and one or two or more of pharmaceutically acceptable surfactants, carriers, diluents, and/or excipients. The pharmaceutically acceptable carriers, diluents, and the like are well known in the medical field and are described in, for example, Remington's Pharmaceutical Sciences, 18th Ed., Mack Publishing Co., Easton, PA (1990), the entire disclosure of which is incorporated herein by reference.

According to an embodiment of the invention, the pharmaceutical composition of the invention can noticeably suppress motility of cancer cells and metastasis of cancer, and for example, the cell motility measured by a scratch assay is markedly suppressed to a level of, for example 1/2, or 1/3 or less. As a result of suppressing motility of cancer cells, the pharmaceutical composition of the invention can reduce the region of metastasis of cancer by, for example, 10%, 20%, 30%, 40%, 50%, 60%, 70%, 80%, 90%, or 90% or more, as compared to a case where an HSP47 inhibitor is not administered.

The cancer targeted by the pharmaceutical composition of the invention is not limited so long as the cancer expresses transcript RNA of HSP47, and examples include sarcomas such as fibrosarcoma, malignant fibrous histiocytoma, liposarcoma, rhabdomyosarcoma, leiomyosarcoma, angiosarcoma, Kaposi's sarcoma, lymphangiosarcoma, synovial sarcoma, chondrosarcoma, and osteosarcoma; carcinomas such as brain tumor, head and neck cancer, breast cancer, lung cancer, esophageal cancer, stomach cancer, duodenal cancer, appendiceal cancer, colorectal cancer, rectal cancer, colon cancer, liver cancer, pancreatic cancer, gallbladder cancer, bile duct cancer, anal cancer, kidney cancer, ureteral cancer, urinary bladder cancer, prostate cancer, penile cancer, testicular cancer, uterine cancer, ovarian cancer, vulvar cancer, vaginal cancer, and skin cancer; leukemia, and malignant lymphoma. Among these, solid tumors are preferable, pancreatic cancer, colorectal cancer, and breast cancer are more preferable, and breast cancer such as triple-negative breast cancer is particularly preferable. Triple-negative breast cancer in particular is a cancer species that highly expresses MYH9 protein and has higher motility than non-triple-negative breast cancer, and the invention can be particularly suitably applied to a cancer species having high motility as in the case of triple-negative breast cancer. Furthermore, the metastatic cancer targeted by the pharmaceutical composition of the invention may be, for example, metastatic lung cancer.

Whether cancer cells express HSP47 can be determined by detecting the expression of transcript RNA of HSP47 at the gene level. Specifically, at the gene level, for example, detection can be made by any known gene expression analysis method such as a Northern blotting method, an RNase protection assay, PCR methods such as RT-PCR and real-time PCR, an in situ hybridization method, or an in vitro transcription method. Furthermore, whether cancer cells express HSP47 can be determined by detecting the expression of HSP47 at the protein level. Specifically, for example, detection can be made by an immunoprecipitation method, EIA (enzyme immunoassay) (for example, ELISA (enzyme-linked immunosorbent assay)), RIA (radioimmunoassay) (for example, IRMA (Immunoradiometric assay), RAST (radioallergosorbent test), and RIST (radioimmunosorbent test)), a Western blotting method, an immunohistochemistry method, an immunocytochemistry method, or a flow cytometry method.

According to an embodiment of the invention, the cells that are subjected to administration of the pharmaceutical composition of the invention are cells in which the transcript RNA of HSP47 is expressed. In normal cells, generally, the transcript RNA of HSP47 is substantially not expressed, except for fibroblasts and myofibroblasts. The cells that are subjected to administration of the pharmaceutical composition of the invention are preferably cancer cells expressing HSP47 protein, and cells expressing MYH9 protein in addition to the HSP47 protein are more preferable. HSP47-positive triple-negative breast cancer also expresses MYH9 protein. Therefore, the pharmaceutical composition of the invention may comprise a MYH9 inhibitor in addition to an HSP47 inhibitor.

According to another embodiment of the invention, the cells that are subjected to administration of the pharmaceutical composition of the invention are triple-negative human breast cancer cells that are negative for hormone receptors (for example, estrogen receptors and progesterone receptors) and negative for human epidermal growth factor receptor (HER2). Diagnosis of triple-negative breast cancer requires pathological examination (mainly immunohistochemical staining) and gene expression analysis of tumor tissue harvested by biopsy. Pathological examination and gene expression analysis of two hormone receptors (an estrogen receptor and a progesterone receptor) and HER2 are carried out, and in a case where the tumor tissue is negative for all of these, the tumor tissue is diagnosed as triple-negative breast cancer. As shown in Fig. 1, cell-cultured triple-negative breast cancer is weakly positive for the expression of HSP47. However, as shown in Fig. 2, triple-negative breast cancer cells have high expression of HSP47 when placed in an in vivo environment. Triple-negative breast cancer, which is a problem in cancer pathology, exhibits increased expression of HSP47 in vivo, and such HSP47-positive triple-negative breast cancer has high metastatic potential.

The pharmaceutical composition of the invention may further comprise another chemotherapeutic agent useful for treating a target disease. Examples of the chemotherapeutic agent include, without limitations, an alkylating agent, an antimetabolite, an antitumor antibiotic substance, an alkaloid, a hormonal therapeutic agent, a platinum complex, an angiogenesis inhibitory agent, a topoisomerase inhibitory agent, and a microtubule agonist.

With regard to the pharmaceutical composition of the invention, in a case where an HSP47 inhibitor is used in combination with another chemotherapeutic agent, these may be included in a single composition, or may be included separately in a plurality of compositions such that those compositions may be separately used or may be used in combination.

According to various embodiments of the invention, the above-described pharmaceutical composition can be used in a state of being carried on various drug delivery carriers . Such carriers are not limited, and examples include polymer nanoparticles, polymer micelles, dendrimers, liposomes, virus nanoparticles, and carbon nanotubes (see Cho K. et al., Clin Cancer Res. 2008 Mar 1;14(5):1310-6, and the like).

The pharmaceutical composition of the invention may be administered by various routes including both oral and parenteral routes, examples of which include, without limitations, routes such as oral, intravenous, intramuscular, subcutaneous, topical, rectal, intratumoral, intraarterial, intraportal, intramedullary, intrapulpal, sublingual, intraoral, intraventricular, transmucosal, transdermal, intranasal, intraperitoneal, intrapulmonary, and intrauterine routes, and the pharmaceutical composition may be formulated into a dosage form appropriate for each of the administration routes. Regarding such a dosage form and preparation method, any known dosage form and method can be appropriately employed (see, for example, Standard Pharmaceutics, edited by Watanabe Yoshiteru, et al., Nankodo, 2003; and Remington's Pharmaceutical Sciences).

Examples of a dosage form appropriate for oral administration include, without limitations, a powder, a granular preparation, a tablet, a capsule, a liquid preparation, a suspension, an emulsion, a gel preparation, and a syrup preparation, and examples of a dosage form appropriate for parenteral administration include injectable preparations such as a solution injectable preparation, a suspension injectable preparation, an emulsion injectable preparation, and a prepared-upon-use type injectable preparation. The preparation for parenteral administration can be in the form of an aqueous or non-aqueous isotonic sterile solution or suspension.

### 3. Kit of invention

The invention also relates to a preparation kit for a metastasis suppressant or a pharmaceutical composition, comprising one or two or more containers that include active ingredients (for example, HSP47 inhibitor), which can be included in the cancer metastasis suppressant or pharmaceutical composition of the invention, singly or in combination; and necessary constituent elements of a cancer metastasis suppressant or a pharmaceutical composition provided in the form of such a kit. The kit according to the invention may comprise, in addition to those described above, instructions in which a preparation method, administration method, and the like for the cancer metastasis suppressant or pharmaceutical composition according to the invention are written, for example, an instruction manual or an electronic recording medium such as a CD or a DVD.

### 4. Method for suppressing metastasis of cancer of invention

An embodiment of the invention relates to a method for suppressing metastasis of cancer, comprising administering an HSP47 inhibitor. According to this method, when a case where an HSP47 inhibitor is not administered is compared with a case where an HSP47 inhibitor is administered, the motility of cancer cells is decreased to, for example, 1/2, or 1/3 or less when measured by a scratch assay. Furthermore, according to the method of the invention, as a result of suppressing the motility of cancer cells, the region of metastasis of cancer can be reduced by, for example, 10%, 20%, 30%, 40%, 50%, 60%, 70%, 80%, 90%, or 90% or more, as compared to the case where an HSP47 inhibitor is not administered.

According to the method of the invention, the patient to be administered is typically a patient who requires cancer treatment. Examples of such a patient include, without limitations, patients who suffer from cancers associated with expression of HSP47 as described above, patients who have been diagnosed to have these cancers, and patients who are at a high risk of developing these cancers. Therefore, the method of the invention may further comprise a step of identifying a patient in need of treatment using the cancer metastasis suppressant or pharmaceutical composition comprising an HSP47 inhibitor of the invention, for example, a step of checking whether a breast cancer patient is a triple-negative breast cancer patient. The step of checking whether a breast cancer patient is a triple-negative breast cancer patient comprises a step of performing pathological examination (mainly immunohistochemical staining) and gene expression analysis of tumor tissue harvested by biopsy, more specifically, a step of performing pathological examination and gene expression analysis of two hormone receptors (an estrogen receptor and a progesterone receptor) and HER2, and in a case where the tumor tissue is negative for all of these, determining that the breast cancer patient has triple-negative breast cancer. Specific examples of the cancer are as described above in connection with the pharmaceutical composition.

With regard to the method of the invention, the pharmaceutical composition of the invention can be used in combination with another active substance or treatment method useful for treating a target cancer. The method of the invention can be used in combination with physical therapies such as radiation therapy and particle beam therapy; surgical therapy such as surgical operation; chemotherapy; molecular targeted therapy; and the like, as the treatment method. The method of the invention can use, as the other active substance, a chemotherapeutic agent such as described above in combination, and examples include an alkylating agent, an antimetabolite, an antitumor antibiotic substance, an alkaloid, a hormonal therapeutic agent, a platinum complex, an angiogenesis inhibitory agent, a topoisomerase inhibitory agent, and a microtubule agonist. In the method of the invention, while general treatments such as cancer cell removal, cell death induction, and cell proliferation suppression are carried out, metastasis of cancer can be suppressed along with these treatments, by employing such a configuration. Therefore, treatment of cancer can be carried out multilaterally by the method of the invention, and the effect of cancer treatment can be enhanced.

An effective amount for the method for suppressing metastasis of cancer of the invention is, for example, an amount that lowers the motility of cancer cells or an amount that reduces the region of metastasis of cancer. Furthermore, an amount that does not cause adverse effects exceeding the benefit provided by administration is preferable. Such an amount can be appropriately determined by an in vitro test using cultured cells or the like, or a test in a model animal such as a mouse, a rat, a dog, or a pig, and such a testing method is well known to those ordinarily skilled in the art. Furthermore, the dosage of a drug used for the treatment method of the invention is known to those ordinarily skilled in the art or can be appropriately determined by the above-described tests or the like.

Specific dosage of the active ingredient (for example, HSP47 inhibitor) that is administered in the method of the invention as described in the present specification can be determined by considering various conditions related to the subject who requires treatment, for example, severity of the symptoms, general health condition of the subject, age, body weight, gender of the subject, diet, timing and frequency of administration, medicine that is being used in combination, responsiveness to treatment, dosage form, and compliance to treatment. The dosage of the active ingredient used for the method of the invention is preferably, in the case of human being, for example, about 0.1 mg to about 1,000 mg per dose, in terms of siRNA molecules per kg of body weight for an adult.

Regarding the route of administration, various routes including both oral and parenteral routes, for example, routes such as oral, intravenous, intramuscular, subcutaneous, topical, intratumoral, rectal, intraarterial, intraportal, intramedullary, intrapulpal, sublingual, intraoral, intraventricular, transmucosal, transdermal, intranasal, intraperitoneal, intrapulmonary, and intrauterine routes are included.

The frequency of administration varies depending on the properties of the agent or composition used, or the conditions of the subject including the above-described items; however, for example, the frequency of administration may be several times a day (that is, 2, 3, 4 times, or 5 or more times, a day), once a day, once every several days (that is, once every 2, 3, 4, 5, 6, or 7 days), once every week, or once every several weeks (that is, once every 2, 3, or 4 weeks).

As used in the present specification, the term "patient" means any biological individual; however, the patient is, for example, an animal, a mammal, or a human individual. According to the invention, the term "patient" typically means a subject who contracts cancer or has a risk of contracting cancer.

Furthermore, the term "treatment" as used in the present specification is meant to include all kinds of medically acceptable prophylactic and/or therapeutic interventions intended for curing, transient remission or prevention of a disease, or the like. For example, the term "treatment" includes medically acceptable interventions for a variety of purposes, including delaying or stopping of the progression of a disease, involution or elimination of lesions, prevention of onset, prevention of recurrence, or the like.

This time, it has been disclosed by the inventors that as the cancer metastasis suppressant or pharmaceutical composition according to the invention suppresses expression of HSP47 (SEQ ID NO:1), the cell motility of triple-negative breast cancer is suppressed, and as a result, metastatic lung cancer can be suppressed.

Therefore, the invention also relates to a cancer metastasis suppressant and a pharmaceutical composition for providing the above-described action, comprising an HSP47 inhibitor; use of an HSP47 inhibitor for the manufacture of a medicine for providing the above-described action; and use of an HSP47 inhibitor for providing the above-described action.

### [Examples]

The invention will be described more specifically by way of the following Examples; however, the technical scope of the invention is not intended to be limited to these Examples.

### Example 1: Acquisition and culture of cell lines

All the human breast cancer cell lines (MDA-MB-468, MDA-MB-231, MDA-MB-157, MCF7, and BT474) used in the present specification were purchased from American Type Culture Collection. These cells were cultured in Dulbecco's Modified Eagle Medium (DMEM, Sigma-Aldrich, St. Louis, MO) supplemented with 10% fetal bovine serum (FBS, Invitrogen Life Technologies).

### Example 2: Expression of HSP47 mRNA and HSP47 protein in human breast cancer cells

Expression of HSP47 mRNA and HSP47 protein in three kinds of triple-negative human breast cancer cells (MDA-MB-468, MDA-MB-231, and MDA-MB-157) and two kinds of non-triple-negative human breast cancer cells (MCF7, BT474) was detected by Real time-PCR and a Western blotting method. The results are shown in Fig. 1.

The expression of mRNA in the three kinds of triple-negative human breast cancer cells was markedly low compared to the expression of mRNA in the two kinds of non-triple-negative human breast cancer cells. Similarly, the expression of HSP47 protein in the three kinds of triple-negative human breast cancer cells was also markedly low compared to the expression of HSP47 protein in the two kinds of non-triple-negative human breast cancer cells.

### Example 3: HSP47-positive cell rate in triple-negative human breast cancer cells

Two kinds of triple-negative human breast cancer cells (MDA-MB-231 and MDA-MB-157) were subcutaneously transplanted into Balb/c nu/nu mice at a concentration of 1×10⁶ cells/50 µL PBS, after tumor formation, tumor cells were harvested, and a flow cytometer analysis was performed using anti-HSP47 antibody (Enzo Life Sciences). The results are presented in Fig. 2. 19.7% of MDA-MB-231 cells expressed HSP47, while 16.7% of MDA-MB-157 cells expressed HSP47.

Therefore, as shown in Fig. 1, cell-cultured triple-negative breast cancer was weakly positive for the expression of HSP47. However, as shown in Fig. 2, triple-negative breast cancer cells had high expression of HSP47 in an in vivo environment.

### Example 4: Cellular proliferative capacity, motility, and lung metastatic potential of HSP47 forced expression triple-negative human breast cancer cells

pCMV6-entry plasmid (Mock) was purchased from OriGene Technologies, Inc. pCMV6-HSP47-Myc plasmid (HSP47:Myc) was purchased from OriGene Technologies, Inc. HSP47 forced expression triple-negative human breast cancer cells (MDA-MB-231 and MDA-MB-157) were created by gene transfer of pCMV6-entry plasmid (Mock) or pCMV6-HSP47-Myc plasmid (HSP47:Myc) .

These cells were seeded on a 6-well plate at a concentration of 1×10⁴ cells/well, and for 5 days after the initiation of culturing, the cellular proliferative capacity was measured by cell counting. The results are shown in Fig. 3. With regard to the MDA-MB-231 cells, in the HSP47 :Myc group, the cellular proliferative capacity increased slightly but insignificantly, compared to the Mock group and the WT group. With regard to the MDA-MB-157 cells, in the HSP47:Myc group, the cellular proliferative capacity significantly increased as compared to the Mock group and the WT group.

Furthermore, these cells were seeded in a double chamber at a concentration of 1×10⁴ cells/well, and on the 24^{th} hour after the initiation of culturing, hematoxylin staining was performed, and the cell motility was measured by cell counting. The results are presented in Fig. 4. With regard to the MDA-MB-231 cells, in the HSP47:Myc group, the cell motility increased to about 3 to 4 times, as compared to the Mock group and the WT group. With regard to the MDA-MB-157 cells, in the HSP47:Myc group, the cell motility increased to about 2 times, as compared to the Mock group and the WT group.

In addition, these cells were subjected to caudal vein administration to Balb/c nu/nu mice at a concentration of 1×10⁶ cells/50 µL PBS, and the metastatic potential to the lung was investigated by harvesting lung tissue on the 30^{th} day after cell administration and performing HE staining. The results are presented in Fig. 5. In the HSP47:Myc group of the MDA-MB-231 cells, remarkably high lung metastasis as compared to the Mock group was observed (stained portion in the middle of the right-side diagram).

### Example 5: Cellular proliferative capacity, motor capacity, and lung metastatic potential of triple-negative human breast cancer cells depending on suppression of expression of HSP47

Control shRNA expression plasmid (shControl) was purchased from OriGene Technologies, Inc. HSP47 shRNA expression plasmid (shHSP47) was purchased from OriGene Technologies, Inc. HSP47 expression-suppressed triple-negative human breast cancer cells (HSP47(+) MDA-MB-231 and HSP47(-) MDA-MB-231) were created by gene transfer of Control shRNA expression plasmid (shControl) or HSP47 shRNA expression plasmid (shHSP47).

These cells were seeded on a 6-well plate at a concentration of 1×10⁴ cells/well, and for 5 days after the initiation of culturing, the cellular proliferative capacity was measured by cell counting. The results are presented in Fig. 6. With regard to the MDA-MB-231 cells, in the shHSP47 group, the cellular proliferative capacity decreased significantly as compared to the shControl group.

Furthermore, these cells were seeded on a 35-mm dish at a concentration of 1×10⁵ cells/well, and on the 24^{th} hour after the initiation of culturing, the cell motility was measured using a scratch assay. The results are presented in Fig. 7. With regard to the MDA-MB-231 cells, in the shHSP47 group, the cell motility significantly decreased to about 1/4 as compared to the shControl group.

In addition, these cells were subjected to caudal vein administration to Balb/c nu/nu mice at a concentration of 1×10⁶ cells/50 µL PBS, and the metastatic potential to the lung was investigated by harvesting lung tissue on the 30^{th} day after the administration of cells and performing HE staining. The results are presented in Fig. 8. With regard to the MDA-MB-231 cells, in the shControl group, lung metastasis was observed (left-hand side of lower left diagram) ; however, in the shHSP47 group, lung metastasis was hardly recognized (lower right diagram). Therefore, it was suggested that suppression of HSP47 expression in cancer cells is effective for suppressing metastasis of cancer.

### Example 6: Interaction between HSP47 and MYH9 in triple-negative breast cancer cells

MDA-MB-231 cells (Mock) and MDA-MB-231 cells expressing HSP47:Myc were washed with ice-cold PBS and were lysed using a lysis buffer (50 mM Tris, 250 mM NaCl, 25 mM EDTA, and 1% NP-40) supplemented with a protease inhibitor (Roche Diagnostics). Immunoprecipitation of HSP47 from the cell lysate was performed at 4°C for 16 hours using anti-Myc immobilized magnetic microbeads (Medical & Biological Laboratories), and the beads were washed using a lysis buffer on a magnetic field. Proteins were eluted from the beads using a 50 mM glycine-HCl buffer (pH 3.5) containing an SDS-PAGE sample buffer. After proteins were eluted using a 0.1 M glycine buffer (pH 2.8), the sample was applied to SDS-PAGE and transferred onto a polyvinylidene difluoride membrane (Millipore, Billerica, MA). After blocking was performed with 5% skim milk in PBS containing 0.05% Tween-20, the membrane was probed with the following primary antibodies: mouse anti-HSP47 monoclonal antibody (Enzo Life Sciences) and mouse anti-MYH9 monoclonal antibody (Elabscience). After being treated with the primary antibodies, the membrane was incubated together with the following secondary antibodies: horseradish peroxidase (HRP)-bound goat anti-rabbit IgG (Cell Signaling Technology) and HRP-bound goat anti-mouse IgG (Cell Signaling Technology). Proteins were detected by a chemiluminescence method using ECL (GE Healthcare, Waukesha, WI).

As a result, no band was observed in the immunoprecipitated Mock group; however, in the immunoprecipitated HSP47:Myc group, respective bands of HSP47 and MYH9 were observed due to the formation of complexes (Fig. 9). Therefore, it was found that HSP47 and MYH9 interact with each other.

### Example 7: Both HSP47 and MYH9 enhance metastatic potential of non-triple-negative breast cancer

In order to determine whether induction of MYH9 enhances the metastatic potential of non-triple-negative breast cancer cells (MCF7) expressing not MYH9 but HSP47, a non-triple-negative breast cancer cell line (Mock + MYH9-GFP) that stably expressed MYH9 was produced (Fig. 10A), and the metastatic potential of Mock + MYH9-GFP cells was investigated. As a plasmid expressing MYH9 (alias: NMIIA), pCMV-NMIIA-GFP plasmid (Origene) was purchased, and this was used. Under the conditions for in vitro culture, the proliferation rate of the Mock + MYH9-GFP cells was slightly higher than that of corresponding Mock cells, in which this plasmid was not introduced (Fig. 10B). In contrast, the tumorigenic potential of the Mock + MYH9-GFP cells was completely different from that of the Mock cells (Fig. 10E). The invasive and migratory capacity of the Mock + MYH9-GFP cells was markedly higher than that of corresponding Mock cells (Figs. 10C and 10D). Furthermore, it was found by a histological analysis that the metastatic potential to the lung of the Mock +MYH9-GFP cells was markedly higher than that of the Mock cells (Fig. 10F). The histological analysis was performed in the same manner as in Example 5.

In addition, MCF7 cells in which the expression of HSP47 was knocked out (KO) (HSP47 KO) were produced as follows (Fig. 10A). HSP47 KO cells were produced using pCG:SapI plasmid containing cloning sites for the Cas9 sequence and the gRNA sequence, which was granted by Dr. Sakurai of Shinshu University (see Sakurai, T. et al. (2014) BMC Biotechnol. 14, 69, and Sakurai, T. et al. (2016) Sci. Rep. 6, 20011) . A gRNA sequence (5'-CAAGATGCGAGACGAGTTATAGG-3' (SEQ ID NO:8)) designed for exon 5 of the human HSP47 genome was inserted into pCG:SapI plasmid. DNA fragments of P2A-mCherry cDNA were amplified using pP2A-mCherry-N1 plasmid (Addgege). The constructed targeting vector and pcDNA3.1(+) (Promega) were co-transfected into MCF7 cell line using Lipofectamine 2000 (Thermo Fisher Scientific). The treated cells were cultured in DMEM supplemented with 10% FBS and G418 (1000 µg/mL, Thermo Fisher Scientific), and cell clones were selected. DNA sequencing of the HSP47 genomic DNA extracted from the cell clones was determined to confirm insertion of the P2A-mCherry gene into a downstream site of the HSP47 gene. Single cell clones were evaluated by a DNA sequence analysis to detect insertions and deletions in the target allele.

The proliferation rate was checked, and it was found that the proliferation rate of the HSP47 KO cells was noticeably lower than that of the Mock cells and the Mock + MYH9-GFP cells (Fig. 10B). The lowering of the proliferation rate of the HSP47 KO cells was not restored even when MYH9 was expressed (Fig. 10B). Furthermore, deletion of HSP47 decreased the tumorigenic potential of MCF7 cells regardless of the expression of MYH9 by the plasmid expressing MYH9 (Fig. 10E) . Enhancement of the motility of MCF7 cells by induction of MYH9 was noticeably suppressed by deletion of HSP47 expression (Fig. 10C and Fig. 10D). Furthermore, the metastatic potential to the lung of MYH9-GFP cells was completely suppressed by deletion of HSP47 expression (Fig. 10F). A histological analysis was performed in the same manner as in Example 5.

These results suggest that expression of both HSP47 and MYH9 is responsible for the metastatic potential of non-triple-negative cells.

### Example 8: In vitro proliferation of triple-negative cells transfected with MYH9 siRNA

Human triple-negative breast cancer cells (MDA-MB-231 and MDA-MB-468) cells were transfected with the following siRNAs using Lipofectamine RNAiMAX (Invitrogen Life Technologies) and cultured for 5 hours at 37°C in the air.

Control siRNA (siControl, Thermo Fisher Scientific, Waltham, MA),
MYH9-A siRNA (siMYH9-A, sense strand: 5'-GGGUAUCAAUGUGACCGAUtt-3' (SEQ ID NO:9); antisense strand: 5'-AUCGGUCACAUUGAUACCCaa-3' (SEQ ID NO:10)),
MYH9-B siRNA (siMYH9-B, sense strand: 5'-CCACCAACCUCACAGAAGAtt-3' (SEQ ID NO:11); antisense strand: 5'-UCUUCUGUGAGGUUGGUGGtg-3' (SEQ ID NO:12)), or
MYH9-C siRNA (siMYH9-C, sense strand: 5'-CGGCAAGGUGGAUUACAAAtt-3' (SEQ ID NO:13); antisense strand: 5'-UUUGUAAUCCACCUUGCCGgc-3'(SEQ ID NO:14))

After 5 hours from the transfection with siRNA, the cells were cultured in Dulbecco's Modified Eagle Medium (DMEM, Sigma-Aldrich, St. Louis, MO) supplemented with 10% fetal bovine serum (FBS, Invitrogen Life Technologies).

Expression of MYH9 protein in the triple-negative human breast cancer cells (MDA-MB-231 and MDA-MB-468) was detected by Real time-PCR in the same manner as in Example 2. The results are presented in Fig. 11A. Expression of MYH9 mRNA in the test groups (siMYH9-A, siMYH9-B, and siMYH9-C) was noticeably low as compared to the expression of MYH9 mRNA in the siControl group.

Furthermore, these cells were seeded on a 6-well plate at a concentration of 1×10⁴ cells/well in the same manner as in Example 5, and for 5 days after the initiation of culturing, the cellular proliferative capacity was measured by cell counting. The results are presented in Fig. 11B. For both of the MDA-MB-231 cells and the MDA-MB-468 cells, the cellular proliferative capacity significantly decreased in the test groups (siMYH9-A, siMYH9-B, and siMYH9-C), as compared to the siControl group.

These cells were seeded on a 35-mm dish at a concentration of 1×10⁵ cells/well, and on the 24^{th} hour after the initiation of culturing, the cell motility was measured using a scratch assay. The results are presented in Fig. 12A. For both the MDA-MB-231 cells and the MDA-MB-468 cells, the cell motility significantly decreased to about 1/5 to 1/3 in the test groups (siMYH9-A, siMYH9-B, and siMYH9-C), as compared to the siControl group.

These cells were cultured in a Transwell chamber at a concentration of 2×10⁴ cells/insert. After 24 hours from the initiation of culturing, cells that passed through a Transwell membrane were immobilized with 4% paraformaldehyde and stained using hematoxylin. The results of the number of cells per field are presented in Fig. 12B. For both the MDA-MB-231 cells and the MDA-MB-468 cells, the cellular invasive capacity significantly decreased to about 1/4 to 1/3 in the test groups (siMYH9-A, siMYH9-B, and siMYH9-C), as compared to the SiControl group (Fig. 12B). Therefore, it was suggested that suppression of MYH9 expression in cancer cells is effective for the suppression of metastasis of cancer.

## Claims

1. A cancer metastasis suppressant, comprising an HSP47 inhibitor.

2. The cancer metastasis suppressant according to claim 1, wherein cancer is breast cancer.

3. The cancer metastasis suppressant according to claim 2, wherein the cancer is triple-negative breast cancer negative for hormone receptors and negative for human epidermal growth factor receptor 2 (HER2).

4. The cancer metastasis suppressant according to claim 3, wherein the hormone receptors are an estrogen receptor and a progesterone receptor.

5. The cancer metastasis suppressant according to claim 3 or 4, wherein the cancer cell is a triple-negative breast cancer cell expressing MYH9 in addition to HSP47.

6. The cancer metastasis suppressant according to any one of claims 1 to 5, wherein the HSP47 inhibitor is an interfering nucleic acid against HSP47, a ribozyme, an antisense nucleic acid, a microRNA, a short hairpin RNA, a vector expressing any of these, or a cell transformed with any of these.

7. A pharmaceutical composition comprising the cancer metastasis suppressant according to any one of claims 1 to 6.

8. The pharmaceutical composition according to claim 7, wherein the pharmaceutical composition is intended for treating cancer.

9. The pharmaceutical composition according to claim 8, wherein the pharmaceutical composition is intended for treating metastatic lung cancer.

10. Use of an HSP47 inhibitor in the manufacture of a medicine for suppressing metastasis of cancer.

11. The use according to claim 10, wherein the cancer is triple-negative breast cancer.

12. A composition for use in the suppression of metastasis of cancer, comprising an HSP47 inhibitor.

13. The composition according to claim 12, wherein the cancer is triple-negative breast cancer.

14. A method for suppressing metastasis of cancer, comprising administering an effective amount of an HSP47 inhibitor to a cancer patient.

15. The method for suppressing metastasis of cancer according to claim 14, wherein the cancer patient is a breast cancer patient, and the method comprises a step of checking whether the breast cancer patient is a triple-negative breast cancer patient before administering the effective amount of the HSP47 inhibitor.

16. The method for suppressing metastasis of cancer according to claim 14 or 15, comprising a step of checking whether the cancer tissue of the cancer patient expresses MYH9 in addition to HSP47, before administering the effective amount of the HSP47 inhibitor.

17. The method for suppressing metastasis of cancer according to any one of claims 14 to 16, wherein the method is carried out together with a therapy selected from the group consisting of surgical therapy, radiation therapy, particle beam therapy, chemotherapy, and molecular targeted therapy.

18. A method for suppressing metastasis of cancer in vitro, comprising treating cancer patient-derived cancer cells in vitro using an effective amount of an HSP47 inhibitor.
